# FASCICULE DE BREVET EUROPEEN

(11) **EP 0 695 345 B1**
(45) Date de publication et mention de la délivrance du brevet: **02.07.1997**
(21) Numéro de dépôt: 94913637.8
(22) Date de dépôt: 13.04.1994
(51) Int. Cl.: C12N 1/20, C02F 3/34, B01D 53/00

(54) **SOUCHE DE MICRO-ORGANISME DU GENRE $i(BACILLUS CEREUS) ET SON UTILISATION**
MIKROORGANISMUS-STAMM DES GENUS BACILLUS CEREUS UND SEINE VERWENDUNG
MICROORGANISM OF GENUS $i(BACILLUS CEREUS) AND USE THEREOF

(30) Priorité: 13.04.1993 FR 9304307
(43) Date de publication de la demande: 07.02.1996
(73) Titulaire: SOCIETE ALSACIENNE D'ALUMINIUM, F-74164 Saint-Julien-en-Genevois Cédex (FR)
(72) Inventeur: PERRY, David, F-31031 Toulouse (FR)
(74) Mandataire: Gillard, Marie-Louise
(86) Numéro de dépôt international: FR9400408
(87) Numéro de publication internationale: WO9424265

(56) Documents cités:
- PROC. INT. BIODEGRADATION SYMP., 3RD 1975 1976 pages 651 - 657 WESLEY O. PIPES 'Biodegradation of taste and odour compounds'
- DATABASE WPI Section Ch, Week 8419, Derwent Publications Ltd., London, GB; Class D16, AN 84-119225 & SU,A,1 033 542 (AS KAZA MICROBIOL) 7 Aoüt 1983
- POLSKIE ARCHIWUM HYDROBIOLOGII vol. 21, no. 1 , 1974 pages 163 - 171 J. MALESZEWSKA 'Degradation of methyl parathion by microorganisms occurring in surface water and sewage'

## Description

La présente invention concerne une nouvelle souche de micro-organisme capable de dégrader des composés organiques et/ou inorganiques, notamment des composés organiques volatils, ci-après dénommés C.O.V.

La destruction des principaux polluants industriels, composés directement toxiques pour l'homme et l'environnement (solvants industriels, hydrocarbures, produits aromatiques) ou polluants impliqués dans les phénomènes géophysiques inquiétants, tels que le réchauffement de la terre (dioxyde de carbone, méthane) ou la formation de trou d'ozone dans la stratosphère (carbures halogénés, méthane, dioxyde de carbone, oxydes de soufre et d'azote), ainsi que des composés malodorants (mercaptans, produits soufrés, ammoniaque, scatols, indoles, etc) fait actuellement l'objet de nombreuses recherches.

Les principales méthodes de destruction sont fonction des concentrations à traiter, de la nature des composés polluants présents, de leur état physico-chimique (effluents gazeux contenant des composés organiques volatils, effluents liquides, boueux ou solides). En ce qui concerne les effluents gazeux, la concentration et la nature des polluants (monocomposés, composés multiples) favorisent l'une ou l'autre solution. En ordre croissant de concentration, on emploie habituellement :
- le traitement biologique
- l'adsorption
- l'absorption
- l'incinération catalytique
- l'incinération thermique
- la condensation.

Ainsi un procédé très utilisé est celui qui consiste à employer de la boue activée qui, en contact avec les effluents gazeux, favorise l'épuration des composés organiques volatils (C.O.V). On notera par exemple que les demandes de brevets EP-A-074 100, EP-A-186 925, et le brevet US 4,723,968 décrivent des procédés de ce type. Il est également possible de mettre en contact des effluents gazeux avec des micro-organismes sélectionnés ; ainsi une souche de Bacillus cereus peut être utilisée pour dégrader des composés malodorants tels que le phénol présents dans des eaux résiduaires (3rd Proc. Int. Biodegrad. Symp., 1975, 651-657). La demande SU-A-1 033 542 décrit l'utilisation d'une autre souche de Bacillus cereus pour réduire la concentration en α-méthylstyrène des effluents industriels. Une autre souche de Bacillus cereus permet de dégrader un pesticide phosphoré tel que le méthylparathion (Polskie Archiwum Hydrobiologii, 1974, 21 (1), 163-174). On peut aussi citer à ce sujet la demande de brevet européen EP-A-100 024 ou bien le brevet US 4,894,162.

On a maintenant trouvé, de manière surprenante, qu'une nouvelle souche de micro-organisme sélectionnée permettait de dégrader de manière efficace de nombreux polluants organiques et inorganiques, seuls ou en combinaisons complexes, contenus dans des effluents gazeux, notamment des effluents gazeux industriels.

Ainsi, l'objet de la présente invention concerne une nouvelle souche de micro-organisme du genre Bacillus cereus, ci-après dénommée souche SAA 1, ainsi que les mutants de cette souche, obtenus par culture de la souche SAA 1 en présence d'agents mutagènes tels que, par exemple, les sulfonates, la NTG (N-méthyl-N'-nitro-N-nitrosoguanidine), etc., ou sous l'action d'un rayonnement énergétique (rayons UV par exemple).

L'invention concerne également les hybrides ou clones issus de la souche SAA 1.

La souche SAA 1 a été prélevée dans la nature, dans un environnement spécifique, puis sélectionnée pour son aptitude à la destruction des C.O.V. Elle a été déposée à la Collection Nationale de Cultures de Micro-organismes (CNCM) le 22 avril 1992 sous le numéro 1-1205.

Cette souche, extrêmement polymorphe, présente successivement sur un même milieu de culture, tel qu'un milieu comprenant du glucose et de l'extrait de levure ou un milieu nutritif standard PCA (Plate Count Agar), les formes bâtonnets (Gram +), les filaments (Gram -), les spores allongées et formées à l'intérieur des bâtonnets ou des filaments, les spores se développant en paquets de 4 à 6 spores par paquets, ou en chaînes de longueur non-limitée. Le développement de ces formes dépend du milieu, de l'âge et des conditions de culture. Du fait du développement asynchrone de ces formes, une même culture peut se révéler Gram + et Gram - simultanément.

Les principales propriétés intrinsèques de cette souche SAA 1 sont les suivantes :
* sa résistance face à de fortcs concentrations de composés organiques volatils ;
* son pouvoir de métabolisation ;
* sa capacité de s'implanter et de se maintenir en environnement complexe aussi bien très faiblement que fortement pollué ;
* sa survie dans un milieu fortement contaminé par d'autres micro-organismes et ce, dans des environnements extrêmes (absence d'aération, interruption de l'alimentation en effluents gazeux pollués).

Ses caractéristiques taxonomiques, déterminées par l'Institut Pasteur à Paris (FRANCE) et par l'Université de Delft (HOLLANDE), sont indiquées respectivement dans les tableaux 1 et 2 ci-dessous :

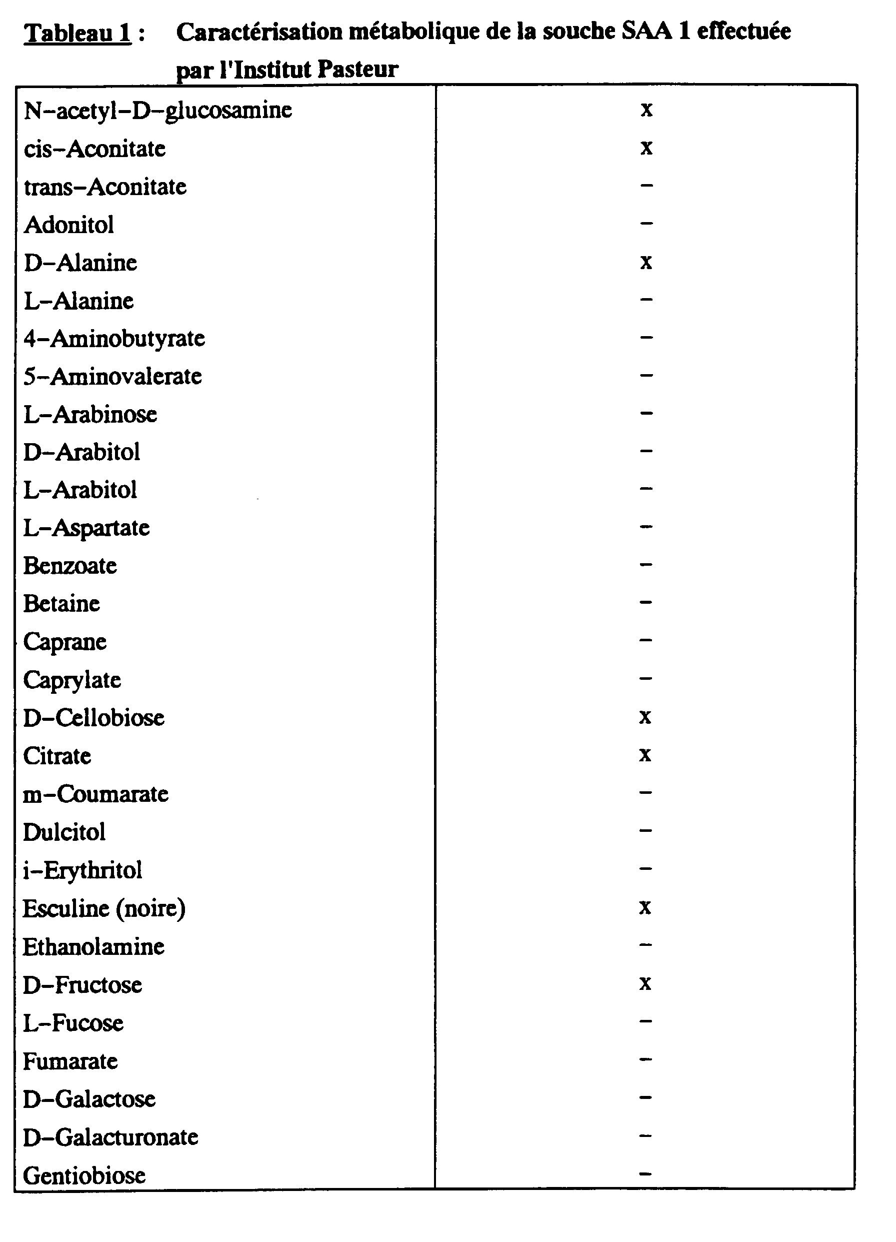

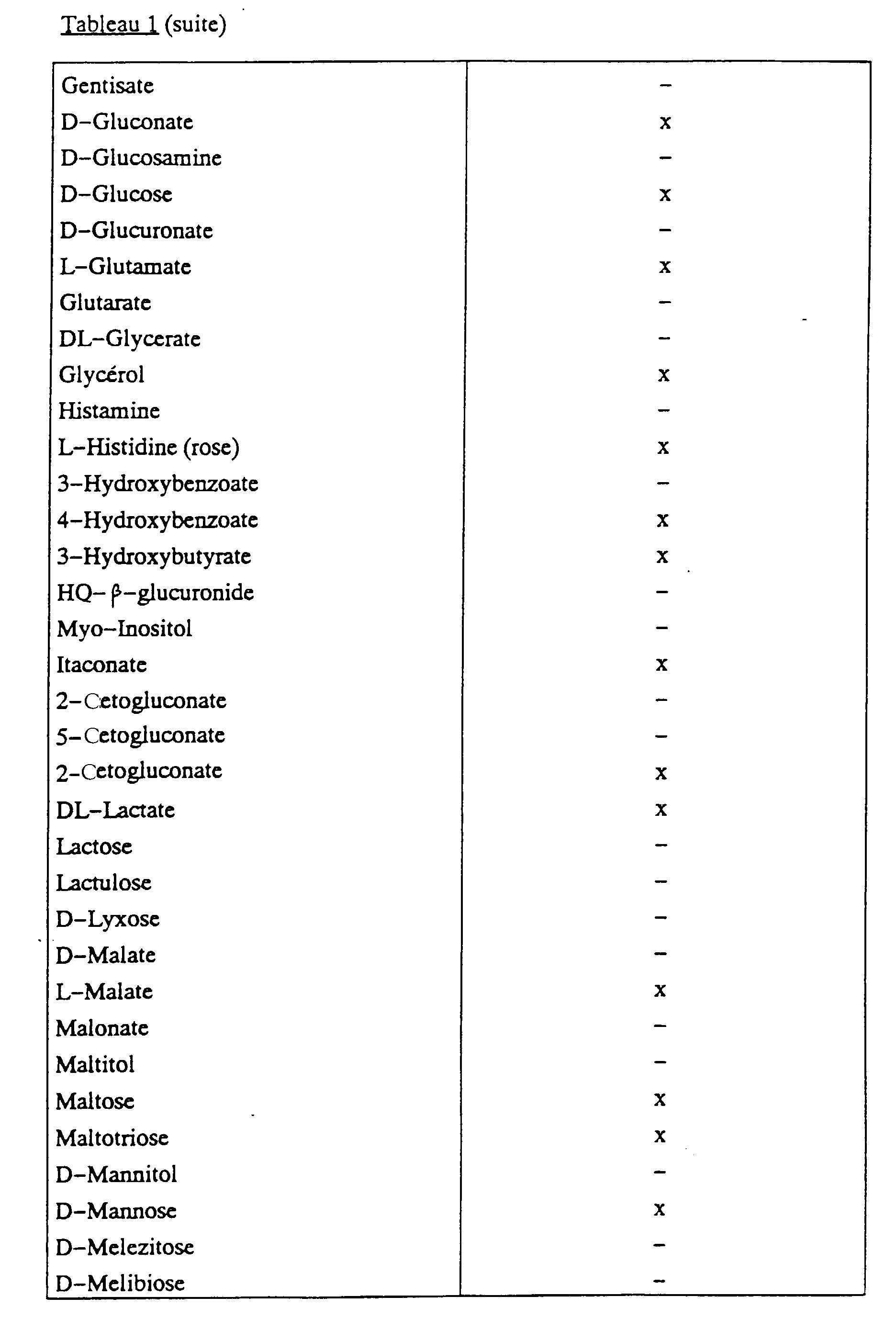

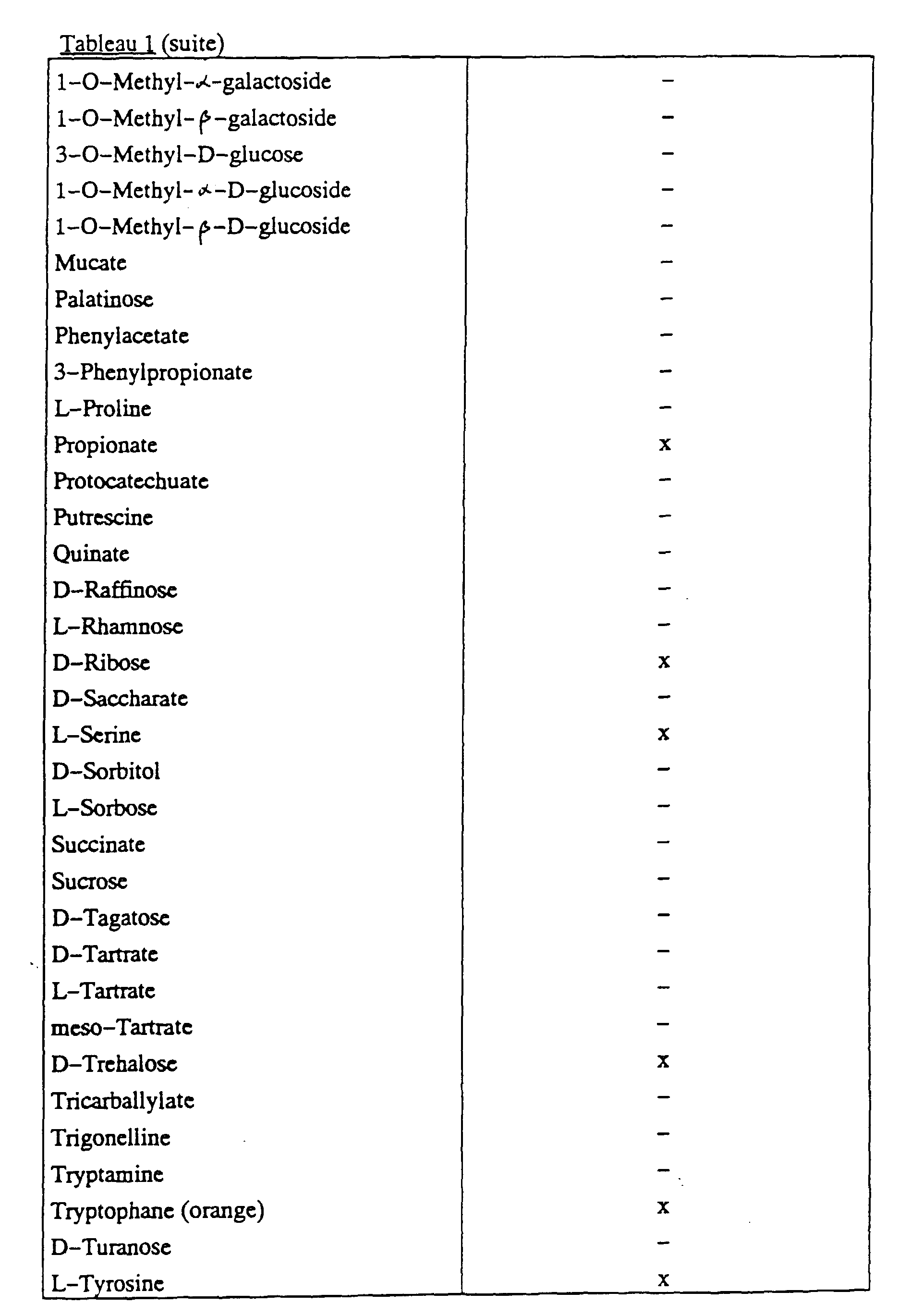

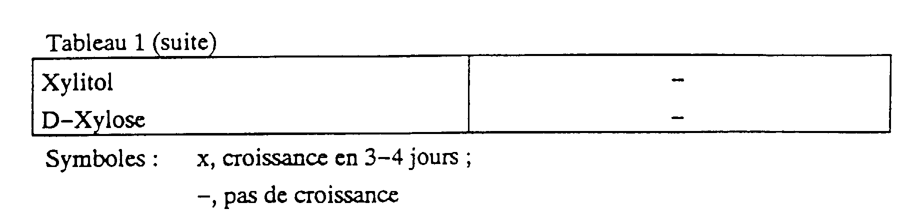

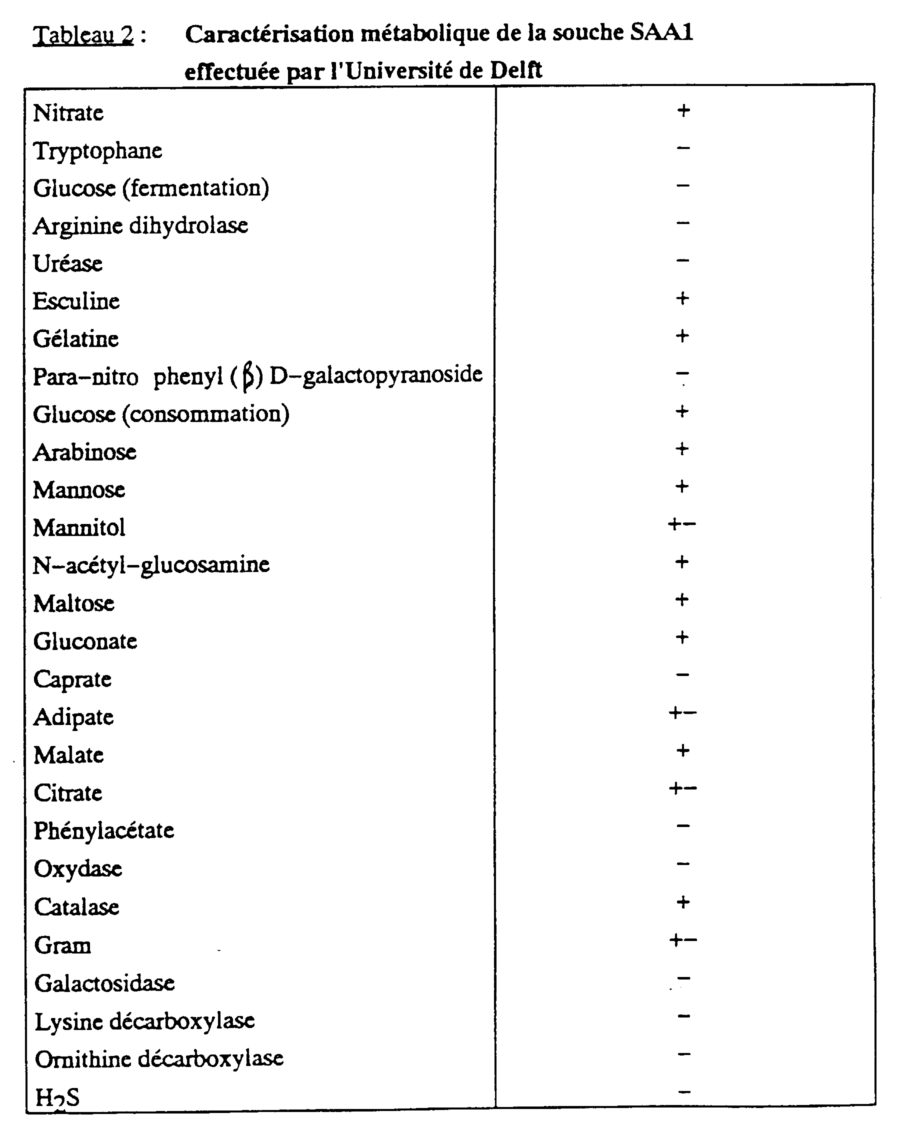

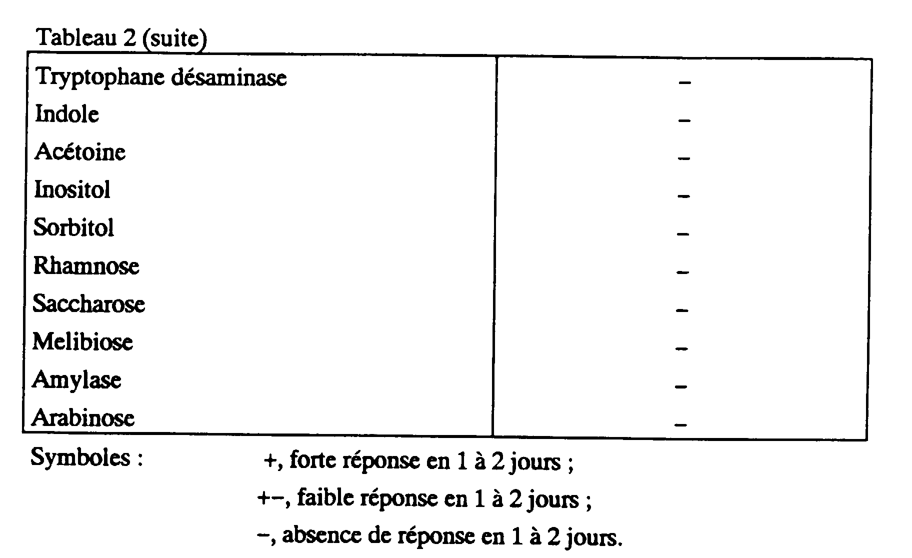

Les caractéristiques génomiques de cette souche ont également été déterminées : des profils de restrictions utiles ont été obtenus avec l'ADN de cette souche clivé avec les endonucléases *Bam*HI, *Bgl*I, *Bgl*II, *Cla*I, *Eco*RV, *Hind*III, *Ml*uI, *Pst*I, *Sma*I, *Sty*I, *Xba*I et hybridé avec la sonde d'ARN ribosomal 16+23S de *Escherichia coli.*

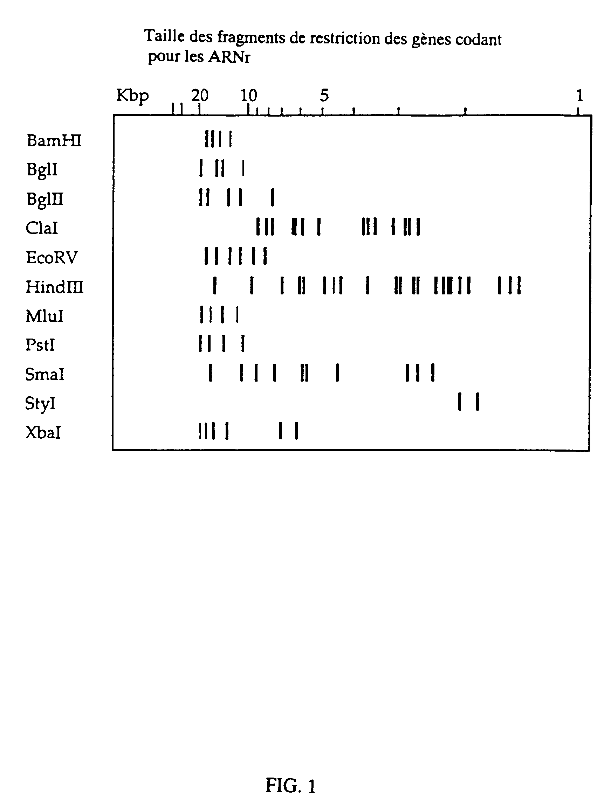

La figure 1 donne la représentation schématique des profils de restriction de gènes codant pour les ARN ribosomaux de la souche SAA 1, obtenus après clivage avec ces onze endonucléases et hybridation avec l'ARNr 16 + 23 S de *Escherichia coli.*

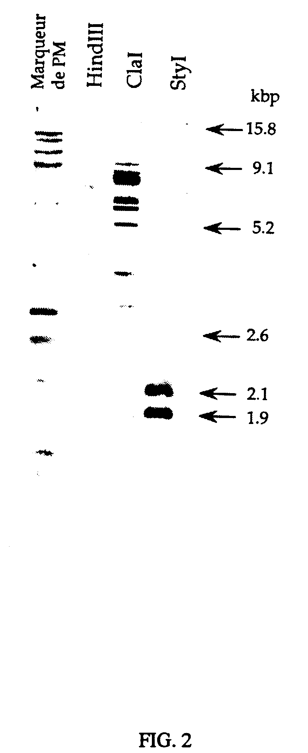

La figure 2 montre les profils (ribotypes) de l'ADN de la souche SAA 1 obtenus après clivage avec les endonucléases *Hind*III, *Cla*I, *Sty*I et hybridation avec l'ARN 16 + 23 S de *Escherichia coli* marqué à l'acétylaminofluorène et détecté par une réaction immuno-enzymatique.

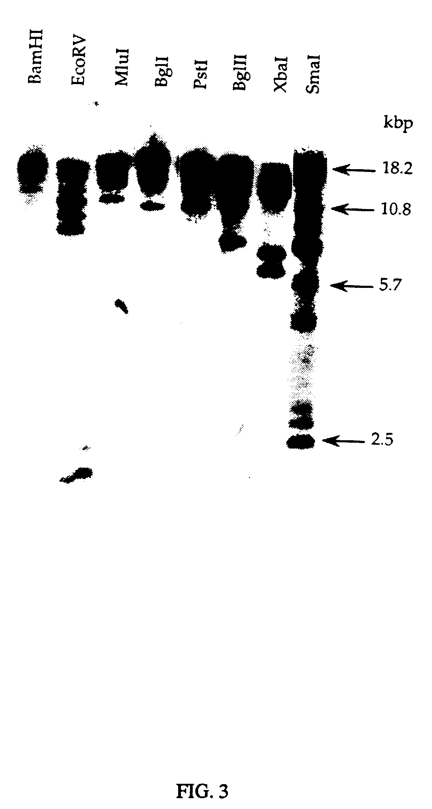

La figure 3 montre les profils (ribotypes) de l'ADN de la souche SAA 1 obtenus après clivage avec les endonucléases *Bam*HI, *Eco*RI, *Mlu*I, *Bgl*I, *Pst*I, *Bgl*II, *Xba*I, *Sma*I et hybridation avec l'ARN 16 + 23 S de *Escherichia coli* marqué par du phosphore radioactif ³²P.

La souche SAA 1 selon l'invention permet de dégrader des composés organiques et/ou inorganiques, en particulier des composés organiques volatils, tels que:
- les alcools, notamment l'éthanol et le propanol ;
- les cétones, notamment la méthyléthylcétone et l'acétone ;
- les éthers, notamment l'éthoxyde de propanol ;
- les acétates, notamment l'acétate d'éthyle ;

On peut aussi utiliser la souche SAA 1 pour dégrader les COV qui sont habituellement utilisés comme solvants, tels que par exemple les solvants aromatiques, les solvants azotés ou les solvants halogénés, ainsi que les aldéhydes, les hydrocarbures, et les résidus de raffinage.

La souche selon l'invention peut également être utilisée pour le traitement des gaz malodorants, tels que l'hydrogène sulfureux ou les mercaptans.

Il est donc possible de dégrader des COV présents dans divers milieux : eau, air, sols, en introduisant directement dans ces milieux la souche SAA 1 cultivée au préalable, ou à l'aide de divers types de réacteurs biologiques. Ces réacteurs peuvent mettre en oeuvre la souche SAA1 suspendue dans un milieu liquide ou bien ladite souche qui circule librement ou qui est retenue à l'aide d'un filtre ou d'une membrane dans le réacteur ; ou encore, ces réacteurs peuvent être agencés pour fournir un support solide sur laquelle la souche SAA 1 se fixe, comme par exemple les réacteurs de type lit fixe ou lit fluidisé. Tout support solide, minéral, organique ou inorganique, naturel ou synthétique, peut convenir : on peut utiliser notamment des matériaux céramiques, des argiles, des roches, des grès ou des matériaux plastiques. De préférence, on utilisera une argile réfractaire, telle que par exemple la chamotte légère.

Les exemples qui suivent illustrent l'invention sans toutefois en limiter la portée :

### Exemple 1: dégradation des COV présents dans de l'eau.

La souche SAA la été cultivée pendant 24 heures sur un milieu de culture contenant du glucose (30 g/l) et de l'extrait de levure (10 g/l).

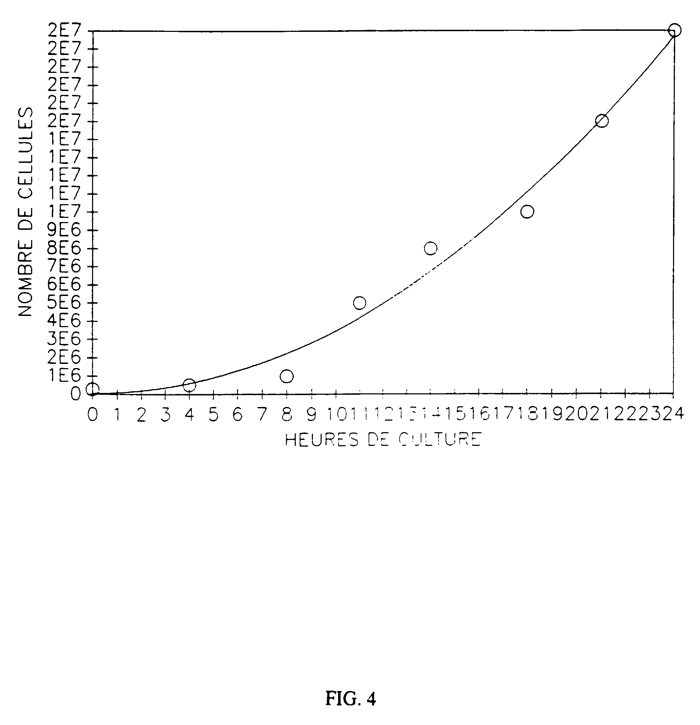

Aux échelles égales ou inférieures à 1 l, la souche SAA 1 est cultivée en milieu liquide agité, dans des fioles d'Erlenmeyer (Tableau 3), et aux échelles supérieures dans des fermenteurs classiques (Tableau 4). Une courbe de croissance typique, représentée sur la Figure 4, sur laquelle on a porté en abscisse le temps de culture et en ordonnée le nombre de cellules, obtenue en fermenteur de 20 l, illustre l'évolution de la culture.

**Tableau 3 :**

| **Milieu liquide et conditions de culture en fioles Erlenmeyer** | |
|---|---|
| Milieu | Glucose 30 g/l d'eau |
| | Extrait de levure 10 g/l d'eau |
| Inoculum | 10 % |
| Température | 30° C |
| Agitation | 50 tpm |
| Aération | Bouchon de coton |
| Durée de culture | 18 à 24 heures |
| D.O. 650 nm | 3,2 à 4 |
| Observation microscopique | Absence de contamination |

**Tableau 4**

| **Milieu liquide et conditions de culture en fermenteur** | |
|---|---|
| Milieu | Glucose 30 g/l d'eau |
| | Extrait de levure 10 g/l d'eau |
| Inoculum | 10 % |
| Température | 30° C |
| Agitation | 400 tours/minute |
| Aération | 0,3 volume(air)/minute/volume (milieu) |
| Durée de culture | 20 à 24 heures |
| D.O 650 nm | 4 à 12 |
| Observation microscopique | Absence de contamination |
| Comptage sur boîte de Petri PCA | ≥ 10⁶ cellules/ml |

La souche SAA 1 a ensuite été mise en présence de 1,10 ou 100 g/l du milieu d'essai ayant la composition suivante :

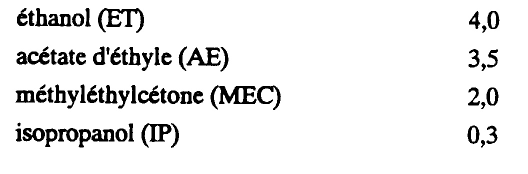

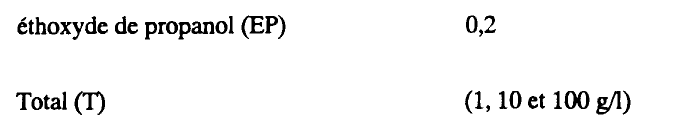

La souche selon l'invention, cultivée dans les conditons indiquées dans le Tableau 3, et mise en présence du milieu d'essai, présente les caractéristiques suivantes :
- efficacité* : 38 %
- productivité : 10,2 g/l/h
- spécificité : dégrade ET, AE, IP, MEC et EP
- biomasse produite : 2 g/l milieu à pH = 6

* l'efficacité correspond au pourcentage de dégradation (COV totaux) au cours de l'essai.

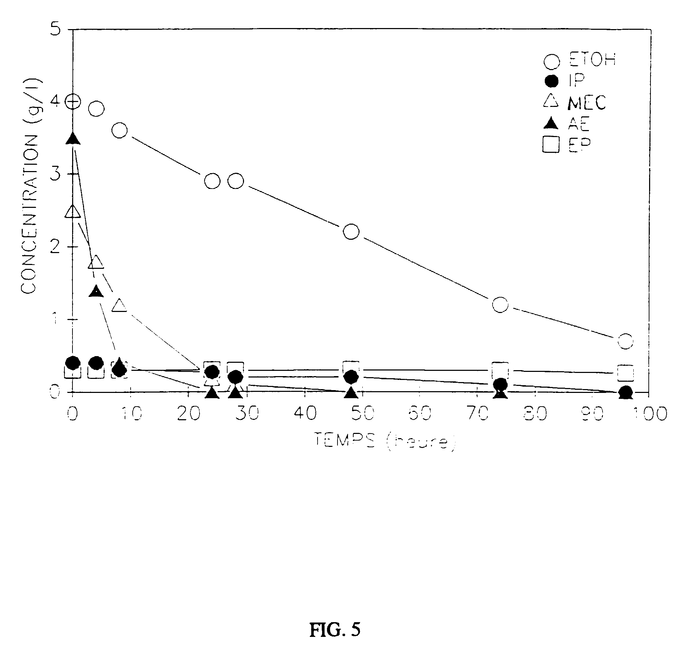

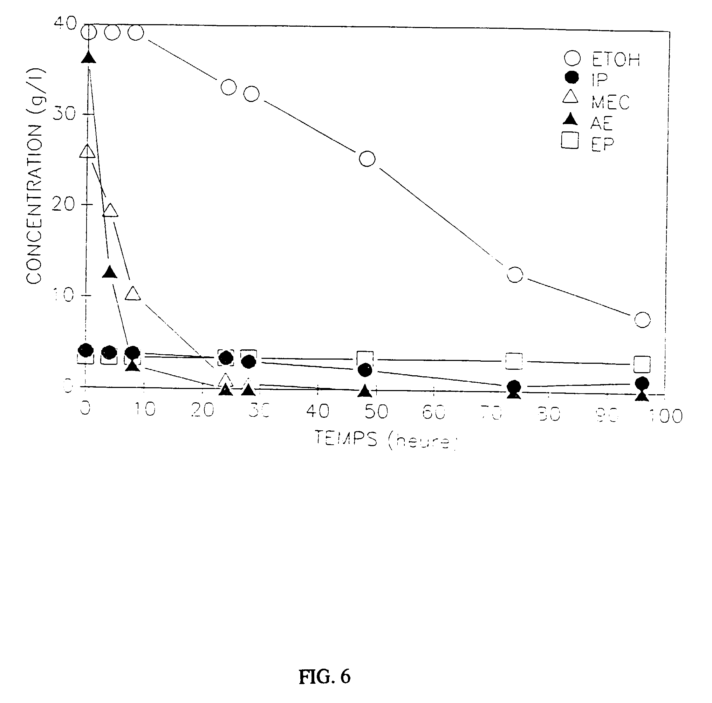

La dégradation des COV par la souche SAA 1, cultivée dans les conditions indiquées dans le Tableau 4 , à partir d'une concentration du milieu d'essai de 10 g/l et 100 g/l est illustrée respectivement par les Figures 5 et 6, qui représentent l'évolution de la concentration des COV du milieu d'essai en fonction du temps.

### Exemple 2: dégradation des COV présents dans des effluents gazeux.

La souche SAA 1 a été cultivée pendant 24 heures sur un milieu de culture identique à celui décrit dans le Tableau 4. Ensuite, la souche est ensemencée à raison de 10 % volume d'inoculum/volume de milieu d'essai contenu dans un réacteur liquide alimenté par un effluent gazeux (contenant le mélange de COV du milieu d'essai de l'exemple 1) par simple bullage à travers un tamis. Les performances de la souche SAA 1 sont indiquées dans le Tableau 5 ci-après :

**Tableau 5 :**

| **performances obtenues avec la souche SAA 1** | | | |
|---|---|---|---|
| | Concentration entrée (mg/m³) | Concentration sortie (mg/m³) | Efficacité (%) |
| Total | 1 186,0 | 19,5 | > 98 |
| ET | 2,1 | < 0,01 | > 99 |
| AE | 1 110,0 | 11,6 | > 98 |
| MEC | 6,5 | 0,35 | > 94 |
| IP | 0,3 | < 0,01 | > 96 |
| EP | 67,8 | 7,6 | > 88 |

La faible quantité de biomasse produite (3,9 exprimée en log cel/ml) pour une efficacité supérieure à 98 % met en évidence les propriétés avantageuses de la souche SAA 1 pour dégrader les COV.

### Exemple 3: Utilisation de la souche SAA 1 dans différents types de réacteurs

On a testé la capacité de la souche SAA 1 à dégrader des COV présents dans les effluents gazeux de machines imprimeuses en injectant ces effluents gazeux dans différents types de réacteurs contenant un support solide, tel que la chamotte légère, sur lequel on dépose la souche SAA 1 pour former un lit bactérien :
- le réacteur 1 (R1) est un réacteur à lit fluidisé contenant 70 l d'eau et 70 kg de chamotte légère ;
- le réacteur 2 (R2) est un réacteur à lit fluidisé multi-étages contenant 50 l d'eau et 50 kg de chamotte légère ;
- le réacteur 3 (R3) est un réacteur à lit fluidisé circulant contenant 50 l d'eau et 50 kg de chamotte légère.

Ce type de réacteurs et leur technique de mise en oeuvre sont classiques et bien connus de l'homme du métier.

La chamotte légère utilisée dans le cas présent a les caractéristiques physiques et chimiques résumées dans le Tableau 6 ci-après :

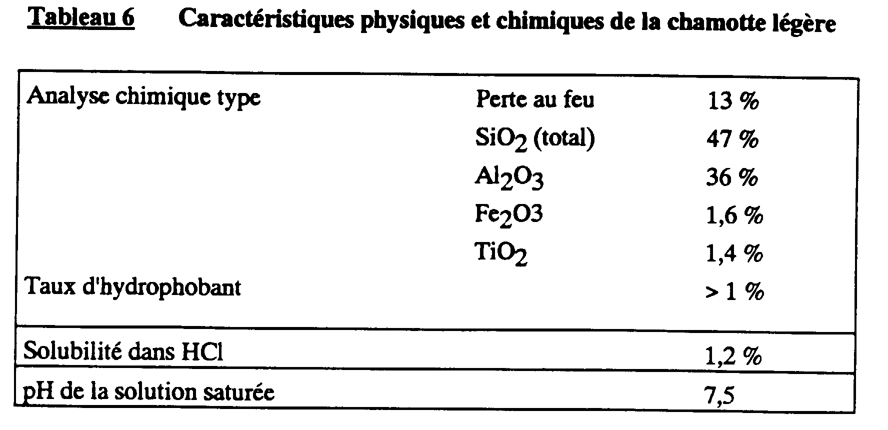

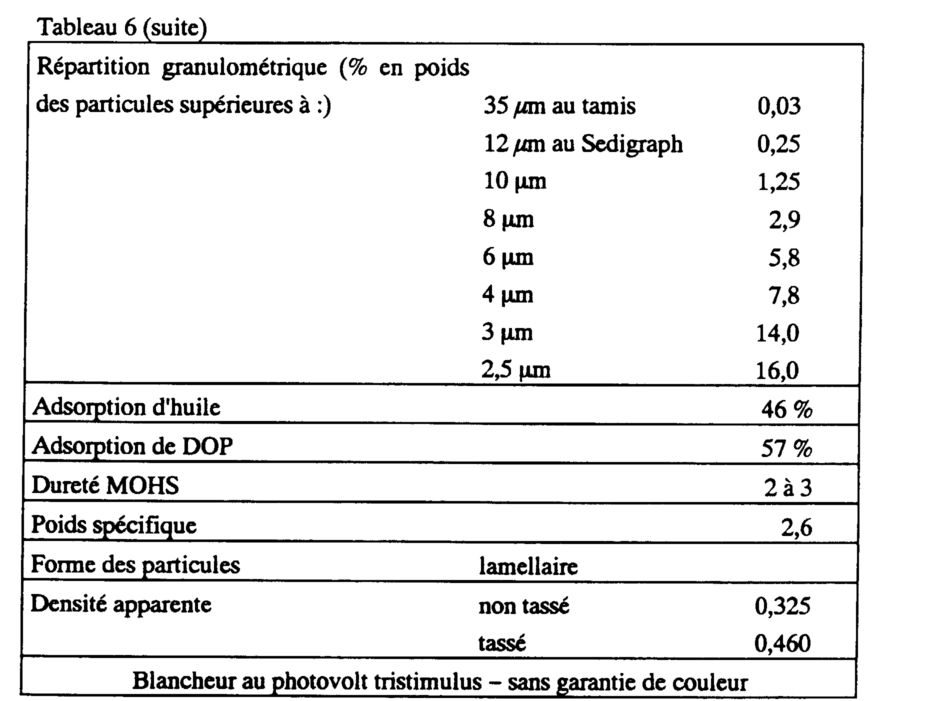

Les réacteurs ont été alimentés avec des effluents gazeux provenant de deux machines imprimeuses, par un système de vannes et de pompes permettant de régler le débit à l'entrée du réacteur.

Les réacteurs sont équipés de sondes de mesure de la température, du pH, de l'oxygène (concentration) et du gaz carbonique (pression) dissous dans la phase liquide à l'intérieur des réacteurs. Les prélèvements ont été effectués sur la phase liquide afin d'analyser les concentrations en composés organiques volatils totales et individuelles. Toutes les mesures de concentrations en composés organiques volatils ont été réalisées à l'aide d'un chromatographe en phase vapeur (CPV).

Le procédé comprend deux étapes successives :
- l'ensemencement du réacteur avec la souche SAA 1; et
- l'alimentation du réacteur avec les effluents gazeux provenant des machines imprimeuses.

La souche SAA 1 a été cultivée dans les conditions indiquées à l'exemple 1 (Tableau 4). L'ensemencement est effectué de manière à établir une concentration initiale de la souche SAA 1 de 10⁵ cellules/ml.

On a constaté que la souche SAA 1 pouvait s'implanter dans un environnement pré-contaminé par l'ensemble des micro-organismes présents dans les effluents gazeux.

Le suivi analytique et microbiologique a été réalisé afin d'évaluer :
- l'efficacité de la souche SAA1, par analyse de la différence entre la concentration des composés organiques volatils à l'entrée et à la sortie du réacteur ;
- la productivité de la souche SAA1 (en mg de composés organiques volatils dégradés/m³ de lit du réacteur/h) - la formation de biomasse et le devenir de la souche SAA 1.

L'ensemble des caractéristiques opérationnelles des différents réacteurs sont rassemblées dans le Tableau 7 :

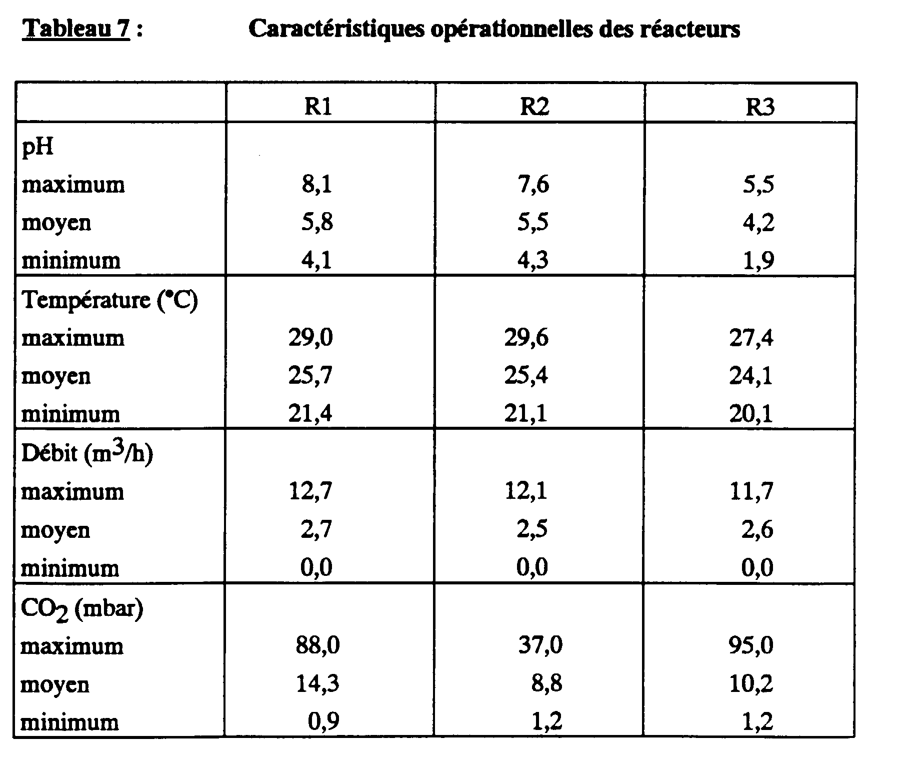

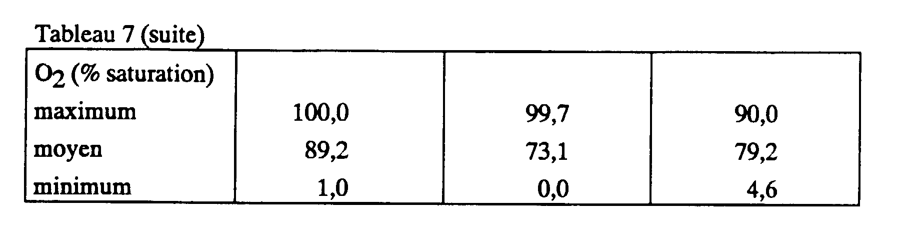

La production d'acide et les fluctuations mises en évidence par le suivi du pH sont les plus faibles dans les réacteurs 1 et 2, avec une moyenne de pH de 5,8 et 5,5 respectivement. L'échange énergétique à l'intérieur de ces deux réacteurs est également légèrement supérieur, puisque la température maximum rencontrée est de 29,0 et 29,6 °C respectivement, par rapport à 27,4 °C dans le réacteur 3.

Les débits moyens d'alimentation en effluents gazeux contenant des COV se situent entre 2,7 et 2,5 m³/heure. La productivité en CO₂, témoin d'une conversion efficace, est la plus élevée dans le réacteur R3, où la concentration d'O₂ à une moyenne de 79,2 % est importante.

Le bilan du traitement donne les éléments ci-dessous, rassemblés dans le Tableau 8 :
* concentration à l'entrée des réacteurs : 1 186 mg de COV total/m³ d'effluents gazeux/heure
   acétate d'éthyle : 1 110 mg/m³,
   éthoxyde de propanol : 67,8 mg/m³,
   méthyléthylcétone : 6,5 mg/m³,
   éthanol: 2,1 mg/m³,
   isopropanol : 0,3 mg/m³.
* dans le milieu liquide à l'intérieur des réacteurs, la concentration reste au minimum dans les réacteurs R1 et R2, à 1,8 et 1,75 g/l respectivement, ce qui est reflété pour tous les COV pris individuellement ;
* à la sortie des réacteurs, les concentrations en COV total, en mg/m³ d'effluent gazeux/heure, sont de 34,8; 42,0 et 46,2 pour les réacteurs 1 à 3 respectivement.

La majorité des COV à la sortie des réacteurs sont l'acétate d'éthyle (23,3 / 30,0 / 33,6 mg/m³ pour les réacteurs R1 à R3 respectivement) et l'éthoxyde de propanol (11,2 / 11,1 / 12,0 mg/m³ respectivement).

**Tableau 8 :**

| **Bilan des réacteurs (valeurs moyennes)** | | | |
|---|---|---|---|
| | R1 | R2 | R3 |
| Concentration entrée (mg/m³) total | 1 186,0 | 1 186,0 | 1 186,0 |
| | | | |
| ET | 2,1 | 2,1 | 2,1 |
| AE | 1 110,0 | 1 110,0 | 1 110,0 |
| MEC | 6,5 | 6,5 | 6,5 |
| IP | 0,3 | 0,3 | 0,3 |
| EP | 67,8 | 67,8 | 67,8 |
| Concentration à l'intérieur (g/l) total | 1,80 | 1,75 | 2,40 |
| | | | |
| ET | 0,20 | 0,20 | 0,40 |
| AE | 0,04 | 0,03 | 0,09 |
| MEC | 0,40 | 0,50 | 0,50 |
| IP | 0,20 | 0,20 | 0,40 |
| EP | 0,90 | 0,80 | 1,00 |
| Concentration sortie (mg/m³) total | 34,8 | 42,0 | 46,2 |
| | | | |
| ET | 0,0 | 0,0 | 0,0 |
| AE | 23,3 | 30,0 | 33,6 |
| MEC | 0,4 | 0,2 | 0,6 |
| IP | 0,0 | 0,0 | 0,0 |
| EP | 11,2 | 11,1 | 12,0 |

Pour ce qui concerne la performance générale des réacteurs (Tableau 9), l'efficacité est de 97,0 %, 96,3 % et 95,9 % respectivement pour les réacteurs R1 à R3. La biomasse moyenne rencontrée dans les réacteurs est de 104 cellules SAA 1, ce qui correspond à une biomasse totale inférieure à 4 g par litre de milieu.

**Tableau 9 :**

| **Performance générale des réacteurs** | | | |
|---|---|---|---|
| | R1 | R2 | R3 |
| Efficacité (%) | 97,0 | 96,3 | 95,9 |
| Biomasse (log cel./ml) | 4,2 | 4 | 3,7 |

Ces résultats montrent que la souche SAA 1 peut être utilisée avec une grande efficacité dans divers types de réacteurs pour dégrader des COV présents dans des effluents gazeux, avec une concentration en COV à la sortie de ces réacteurs inférieure à 50 mg/m³, soit un niveau bien inférieur à la valeur limite fixée par la législation européenne (150 mg/m³).

Il est donc possible d'utiliser la souche SAA 1 selon l'invention dans les industries qui produisent ou mettent en oeuvre des composés organiques volatils, par exemple les imprimeries, mais aussi les stations d'épuration d'eau ou encore les décharges collectives et installations d'hydrocarbures (cuves et réservoir, colonnes de distillation, etc.).

## Revendications

1. Micro-organisme du genre Bacillus cereus, déposé à la CNCM sous le numéro I-1205 et ses mutants capables de dégrader les composés organiques et/ou inorganiques.

2. Utilisation du micro-organisme selon la revendication 1 pour dégrader des composés organiques et/ou inorganiques.

3. Utilisation selon la revendication 2, caractérisée en ce que les composés organiques sont des composés organiques volatils.

4. Utilisation selon la revendication 3, caractérisée en ce que les composés organiques volatils sont choisis parmi l'éthanol, l'acétate d'éthyle, la méthyléthylcétone, l'acétone, l'isopropanol ou l'éthoxyde de propanol.

## Patentansprüche

1. Mikroorganismus der Art Bacillus cereus, der bei der Hinterlegungsstelle CNCM unter der Nummer I-1205 hinterlegt wurde, und seine Mutanten, die dazu befähigt sind, organische Verbindungen und/oder anorganische Verbindungen abzubauen.

2. Verwendung des Mikroorganismus nach Anspruch 1, um organische und/oder anorganische Verbindungen abzubauen.

3. Verwendung nach Anspruch 2,
dadurch gekennzeichnet, daß
die organischen Verbindungen flüchtige organische Verbindungen sind.

4. Verwendung nach Anspruch 3,
dadurch gekennzeichnet, daß
die flüchtigen organischen Verbindungen unter Ethanol, Ethylacetat, Methylethylketon, Aceton, Isopropanol oder Ethoxypropanol ausgewählt sind.

## Claims

1. A microorganism of the genus Bacillus cereus, deposited at the CNCM, number I-1205, and mutants thereof, which can degrade organic and/or inorganic compounds.

2. The use of a microorganism according to claim 1 to degrade organic and/or inorganic compounds.

3. The use according to claim 2, characterized in that the organic compounds are volatile organic compounds.

4. The use according to claim 3, characterized in that the volatile organic compounds are selected from ethanol, ethyl acetate, methylethylketone, acetone, isopropanol, and ethoxypropanol.
